(19) **Europäisches Patentamt European Patent Office Office européen des brevets**

(11) **EP 3 639 735 B1**

(12) **FASCICULE DE BREVET EUROPEEN**

(45) Date de publication et mention de la délivrance du brevet:
**11.01.2023 Bulletin 2023/02**

(21) Numéro de dépôt: **19197438.5**

(22) Date de dépôt: **16.09.2019**

(51) Classification Internationale des Brevets (IPC):
**A61H 33/14** (2006.01)     **A61B 5/00** (2006.01)
**A61B 5/08** (2006.01)     **A61H 31/00** (2006.01)

(52) Classification Coopérative des Brevets (CPC):
**A61B 5/72; A61B 5/082; A61B 5/4848;**
**A61B 5/742; A61H 31/005; A61H 31/006;**
**A61M 16/0051; A61M 16/0066; A61M 16/024;**
A61H 2033/145; A61H 2201/5007;
A61H 2201/5043; A61H 2230/206; A61M 16/0069;
A61M 16/0833;     (Cont.)

(54) **APPAREIL DE MONITORAGE OU DE VENTILATION POUR RÉANIMATION CARDIO-PULMONAIRE AVEC DÉTERMINATION D'UN INDICE D'OUVERTURE DES VOIES RESPIRATOIRES**

ÜBERWACHUNGS- ODER BEATMUNGSGERÄT FÜR DIE KARDIOPULMONARE REANIMATION MIT BESTIMMUNG EINES ÖFFNUNGSINDEXES DER ATEMWEGE

MONITORING OR VENTILATION DEVICE FOR CARDIOPULMONARY RESUSCITATION WITH DETERMINATION OF AN INDEX OF OPENING OF THE AIRWAYS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **15.10.2018 FR 1859509**

(43) Date de publication de la demande:
**22.04.2020 Bulletin 2020/17**

(73) Titulaire: **Air Liquide Medical Systems**
**92182 Antony Cedex (FR)**

(72) Inventeurs:
• **RICHARD, Jean-Christophe**
**92182 ANTONY Cedex (FR)**
• **RIGOLLOT, Marceau**
**92182 ANTONY Cedex (FR)**
• **BADAT, Bilal**
**92182 ANTONY Cedex (FR)**

(74) Mandataire: **Air Liquide**
**L'Air Liquide S.A.**
**Direction de la Propriété Intellectuelle**
**75, Quai d'Orsay**
**75321 Paris Cedex 07 (FR)**

(56) Documents cités:
**WO-A1-2014/072981     WO-A2-2010/059049**
**US-A1- 2016 287 170**

(52) Classification Coopérative des Brevets (CPC):
(Cont.)
A61M 16/085; A61M 2205/05; A61M 2205/505;
A61M 2205/52; A61M 2205/581; A61M 2205/583;
A61M 2205/584; A61M 2205/8212; A61M 2230/04;
A61M 2230/205; A61M 2230/30; A61M 2230/432;
A61M 2230/50

**Description**

L'invention porte sur un appareil de monitorage et/ou d'assistance respiratoire utilisable pendant une réanimation cardio-pulmonaire (RCP) comprenant des compressions thoraciques successives réalisées sur un patient, en particulier un ventilateur médical servant à fournir un gaz respiratoire au patient pendant la RCP, c'est-à-dire un patient en arrêt cardiaque soumis à un massage cardiaque avec alternance de compressions ou contractions thoraciques et de relâchements, avec détermination et affichage de l'indice AOI (i.e., *Airways Opening Index*), c'est-à-dire l'indice d'ouverture des voies respiratoires du patient, ou d'un indice AOI moyen.

Les appareils médicaux de ventilation mécanique, encore appelés appareil d'assistance respiratoire ou ventilateurs médicaux, sont couramment utilisés pour fournir du gaz respiratoire, par exemple de l'air enrichi ou non en oxygène, à certains patients souffrant de troubles respiratoires. La fourniture du gaz respiratoire au patient est couramment opérée au moyen d'une micro-soufflante (aussi appelée turbine ou compresseur) motorisée et pilotée, comme notamment décrit par EP-A-3093498, EP-A-2947328, EP-A-2986856, EP-A-2954213 ou EP-A-2102504.

Il est connu de monitorer les composés gazeux présents dans le gaz administré aux patients que ce soit avec un appareil de monitorage ou avec un ventilateur équipé d'un afficheur. En particulier, il peut être très utile de suivre le $CO_2$ résultant des échanges gazeux pulmonaires, c'est-à-dire le $CO_2$ produit par le métabolisme du patient, transporté jusqu'aux poumons grâce à la circulation sanguine puis évacué lors de l'expiration du patient. Ainsi, l'etCO$_2$ pour end tidal $CO_2$ ou $CO_2$ en fin d'expiration, correspond à la mesure de la fraction de $CO_2$ expirée dans les gaz recueillis lors de l'expiration d'un individu, que son inspiration soit naturelle ou assistée, c'est-à-dire obtenue par ventilation mécanique. Le document US-A-2016/0287170 enseigne un tel monitorage de l'etCO$_2$.

Pendant une réanimation cardio-pulmonaire ou RCP pratiquée sur une personne en arrêt cardio-pulmonaire, avec mise en œuvre d'un massage cardiaque, le $CO_2$ alvéolaire, qui dépend non seulement des rapports ventilation/perfusion pulmonaire mais aussi de la quantité de $CO_2$ généré par le métabolisme cellulaire, représente un paramètre très utile au secouriste, par exemple un médecin, pour juger de l'efficacité de la RCP.

En théorie, plus la RCP est efficace, plus le débit cardiaque généré par les compressions thoraciques est important, plus la quantité de $CO_2$ ramenée vers les poumons est importante.

De ce fait, le monitorage de l'etCO$_2$, qui reflète indirectement le $CO_2$ alvéolaire, est de plus en plus utilisé pour opérer un suivi de la RCP de façon non invasive, c'est-à-dire pour informer et aider le secouriste pendant qu'il opère le massage cardiaque, i.e., alternance de compressions thoraciques (CT) et de relâchements.

La représentation graphique des variations de teneur en $CO_2$ dans les gaz respiratoires d'un patient au fil du temps (en secondes) est appelée capnogramme.

Lors d'une RCP sur un patient en arrêt cardio-respiratoire (ACR), le capnogramme est très différent de celui obtenu sur patient qui n'est pas en ACR, pour plusieurs raisons, notamment :

- Les CTs génèrent des déplacements de petits volumes de gaz qui perturbent le capnogramme entre deux cycles ventilatoires. On observe ainsi souvent des tracés oscillants étant donné que la valeur maximale de $CO_2$ sur chaque CT ne cesse de varier.
- Les rapports ventilation/perfusion qui sont le reflet de la physiologie respiratoire sont très fortement modifiés. De plus, les petits tronçons de gaz mobilisés par les CTs peuvent passer plusieurs fois devant le capteur. La concentration maximale observée, lors de chaque CT, est donc souvent éloignée de la véritable concentration alvéolaire.
- Des comportements dynamiques d'ouverture et de fermeture des petites voies aériennes ont été rapportés. Ce phénomène compromet les échanges gazeux et donc l'interprétation des concentrations de $CO_2$ lors de la RCP. Plus précisément, au cours d'une RCP, les petites voies aériennes intra-pulmonaires peuvent se fermer modifiant ainsi le signal de $CO_2$ expiré vu par la sonde de capnographie

On comprend donc que l'etCO$_2$, tel qu'il est mesuré actuellement, c'est-à-dire lors de chaque compression (aussi appelée contraction) thoracique (CT), ne permet pas d'obtenir une approximation fiable du $CO_2$ alvéolaire, alors que le $CO_2$ alvéolaire est important car il peut être le reflet de la qualité de la RCP, donc du massage.

Dit autrement, opérer une mesure et un monitorage du $CO_2$ sans prise en compte de tout ou partie de ces facteurs, en particulier de l'impact de la ventilation réalisée sur le patient en arrêt cardiaque et de la variabilité du signal de $CO_2$ entre deux cycles machine, rend tout exploitation de la mesure de $CO_2$ peu fiable, voire inutilisable.

Par ailleurs, les solutions actuelles de monitorage de l'etCO$_2$ sont adaptées aux variations de $CO_2$ provoquées par la respiration, qu'elle soit mécanique ou spontanée. Les fréquences en jeu sont de l'ordre de 10 à 40 c/min. Les algorithmes et mécanismes mis en œuvre sont adaptés à ces fréquences et à des variations faibles du $CO_2$ entre deux respirations du patient.

Or, pendant une RCP, les fréquences des CTs sont proches de 100 c/min, les volumes de gaz mobilisés faibles et les débits de gaz importants et irréguliers. De plus, le problème d'espace mort évoqué précédemment s'ajoute à ces difficultés puisque, du fait des CTs, une même fraction de gaz peut être analysée plusieurs fois par le capteur de $CO_2$,

à défaut de mise en place d'un rinçage ou purge de l'espace mort.

Dans ces conditions, la valeur de l'etCO$_2$ affichée par les ventilateurs ou moniteurs actuels est rafraîchie à une fréquence inadéquate puisque ceux-ci tentent de suivre l'évolution du CO$_2$ à la fréquence du massage, à savoir 100 c/min. En d'autres termes, les valeurs d'etCO$_2$ affichées par les ventilateurs ou moniteurs actuels ne sont pas représentatives d'une concentration de CO$_2$ liée au métabolisme du patient car l'origine du gaz analysé n'est pas garantie, c'est-à-dire que les valeurs mesurées actuellement sont souvent erronées car elles ne reflètent pas ou alors que très mal, la concentration en CO$_2$ alvéolaire.

On peut citer les documents WO-A-14072981, US-A-2016/133160 et US-A-2012/016279, qui proposent des méthodes de suivi de la teneur en CO$_2$ dans les gaz expirés par un patient subissant une RCP.

Le problème dès lors est de proposer un appareil de monitorage et/ou d'assistance respiratoire amélioré, tel qu'un moniteur ou un ventilateur, utilisable pendant une réanimation cardio-pulmonaire (RCP) avec massage cardiaque comprenant des compressions thoraciques (CTs) de durée (dt) réalisées sur le patient et des relâchements successifs, qui permette d'informer en temps réel l'équipe de secours de l'ouverture ou non des voies aériennes et donc si les CTs successives opérées génèrent du débit ventilatoire ou non.

La solution de l'invention concerne alors un appareil de monitorage et/ou d'assistance respiratoire utilisable pendant une réanimation cardio-pulmonaire (RCP) comprenant des compressions thoraciques (CT) successives de durée (dt) réalisées sur le patient et des relâchements, en particulier un appareil choisi parmi les appareils de ventilation assistée comprenant une source de gaz respiratoire, les moniteurs cardiaques et les moniteurs/défibrillateurs cardiaques, comprenant :

- des moyens de mesure de teneur en CO$_2$ pour opérer des mesures de concentration en CO$_2$ produit par le patient pendant la réanimation cardio-pulmonaire (RCP), et fournir des signaux de mesure de teneur en CO$_2$ à des moyens de traitement de signal,
- des moyens de traitement de signal configurés pour traiter les signaux de mesure de teneur en CO$_2$ provenant des moyens de mesure de teneur en CO$_2$, et
- au moins une interface graphique utilisateur (IGU),

caractérisé en ce que les moyens de traitement de signal sont configurés pour :

a) déterminer au moins une valeur de teneur maximale en CO$_2$ (Vmax) et au moins une valeur de teneur minimale en CO$_2$ (Vmin), pendant au moins une durée (dt) d'au moins une compression thoracique, et
b) calculer au moins :

. un indice AOI d'ouverture des voies respiratoires (i.e., *Airways Opening Index*) tel que : AOI = (Vmax-Vmin) / Vmax,
ou
. un indice moyen AOI$_{moy}$ à partir de plusieurs indices AOI d'ouverture successifs obtenus pendant les durées (dt) de n compressions thoraciques (avec n>1) successives, et

c) transmettre à ladite interface graphique utilisateur, au moins une valeur d'indice AOI ou au moins une valeur d'indice moyen AOI$_{moy}$, et

- l'interface graphique utilisateur est configurée pour afficher ladite au moins une valeur d'indice AOI ou d'indice moyen AOI$_{moy}$, ou une représentation graphique de ladite au moins une valeur d'indice AOI ou d'indice moyen AOI$_{moy}$.

En d'autres termes, selon l'invention, il est proposé d'analyser les variations de teneurs en CO$_2$ pendant les CTs, d'en extraire les teneurs maximale (Vmax) et minimale (Vmin) en CO$_2$ et de les utiliser pour calculer un indice d'ouverture des voies aériennes ou indice AOI (pour *Airways Opening Index*) qui est représentatif de la qualité du massage cardiaque et du niveau d'ouverture des voies aériennes (alvéoles, bronchioles, etc.), ce qui permet d'avoir une meilleure estimation de la quantité et de la qualité de ventilation de ces voies aériennes. Cet indice AOI et/ou un indice moyen AOI$_{moy}$ est ensuite affiché sur une IGU soit sous forme d'une valeur numérique, par exemple un %, soit sous forme d'une représentation graphique, tel qu'une courbe ou préférentiellement d'un pictogramme ou analogue.

Par exemple, l'indice AOI est, pendant une CT, égal :

- à 1 (soit 100%) lorsque la teneur minimale Vmin en CO$_2$ est nulle CO$_2$ (i.e. 0 mm Hg), ce qui correspond à des voies aériennes totalement ouvertes chez le patient, et
- à 0 (soit 0%) lorsqu'il n'y a aucune variation de CO$_2$, c'est-à-dire lorsque Vmax est égale à Vmin. Ceci correspond à des voies aériennes fermées.

- entre 0 et 1, pour les teneurs minimales Vmin en $CO_2$ intermédiaires, correspondant à des voies aériennes plus ou moins ouvertes ou fermées.

On comprend immédiatement que connaître cet indice AOI, donc pouvoir connaître, en temps réel, l'état des voies aériennes du patient qu'il est en train de traiter, est d'une grande utilité pour le secouriste pratiquant la RCP. Utiliser un affichage de type pictogramme (i.e. dessin, icone ou analogue) est particulièrement intéressant car il permet à l'utilisateur d'avoir une vision simple et immédiate de l'état des voies aériennes du patient.

Il est à noter que, par convention dans le médical, les teneurs en $CO_2$ sont exprimées sous forme de pression partielle de $CO_2$, c'est-à-dire préférentiellement en mmHg, ou en kPa ; toutefois, elles pourraient aussi être exprimées en une autre unité (e.g. % en volume, % molaire....).

Selon le cas, l'appareil de monitorage et/ou d'assistance respiratoire selon l'invention peut comprendre l'une ou plusieurs des caractéristiques techniques suivantes :

- les moyens de traitement de signal sont configurés pour :

  i) déterminer plusieurs valeurs de teneur maximale en $CO_2$ (Vmax) et plusieurs valeurs de teneur minimale en $CO_2$ (Vmin), pendant les durées (dt) de plusieurs compressions (i.e. contractions) thoraciques successives,
  ii) calculer les indices AOI d'ouverture successifs correspondants auxdites plusieurs valeurs de teneur maximale en $CO_2$ (Vmax) et plusieurs valeurs de teneur minimale en $CO_2$ (Vmin) et
  iii) calculer un indice moyen $AOI_{moy}$ à partir des indices AOI d'ouverture successifs obtenus pour les n compressions thoraciques.

- les moyens de traitement de signal sont configurés pour calculer un indice moyen $AOI_{moy}$ à partir des indices AOI d'ouverture successifs obtenus pour n compressions thoraciques, tel que :

$$AOI_{moy} = \sum_{i=1}^{n} AOI(i)\, /n$$

où : n est un nombre entier de CT, avec n > 1.
- de préférence, les moyens de traitement de signal sont configurés pour déterminer les valeurs de teneur maximale en $CO_2$ (Vmax) et de teneur minimale en $CO_2$ (Vmin) pendant la durée (dt) de chaque CT.
- les moyens de traitement de signal sont configurés pour transmettre à l'IGU, au moins une valeur d'indice AOI ou au moins une valeur d'indice moyen $AOI_{moy}$ calculée, et l'interface graphique utilisateur est configurée pour afficher ladite au moins une valeur d'indice AOI ou d'indice moyen $AOI_{moy}$.
- l'IGU est configurée pour afficher au moins une valeur d'indice AOI ou d'indice moyen $AOI_{moy}$ sous forme d'une valeur numérique, en particulier une valeur exprimée en pourcentage (%).
- de façon alternative, l'IGU est configurée pour afficher au moins une valeur d'indice AOI ou d'indice moyen $AOI_{moy}$ sous forme d'une représentation graphique, par exemple un pictogramme, une courbe, un barre-graphe, un « camembert »... ou toute autre représentation.
- de préférence, l'IGU est configurée pour afficher au moins une valeur d'indice AOI ou d'indice moyen $AOI_{moy}$ sous forme d'au moins un pictogramme, c'est-à-dire un dessin, une icone ou analogue. Par exemple, le pictogramme peut représenter des poumons de taille variable/différente en fonction de la valeur d'indice AOI ou d'indice moyen $AOI_{moy}$. Dans cet exemple, plus la valeur d'indice AOI ou d'indice moyen $AOI_{moy}$ est élevée, donc représentative d'une ouverture importante des voies aériennes du patient, plus le pictogramme affiché (i.e. poumons) aura une taille (i.e. dimension) importante sur l'IGU, et inversement. On peut aussi prévoir un affichage du pictogramme en différentes couleurs en fonction de ladite valeur d'indice AOI ou $AOI_{moy}$, voire les deux, à savoir des tailles et couleurs différentes en fonction de l'indice.
- les moyens de mesure de teneur en $CO_2$ comprennent un capnomètre ou tout autre capteur de $CO_2$.
- les moyens de mesure de teneur en $CO_2$ sont configurés pour opérer des mesures de teneur en $CO_2$ en continu.
- les moyens de traitement de signal comprennent au moins un microprocesseur, en particulier un microcontrôleur, mettant en œuvre au moins un algorithme.
- les moyens de traitement de signal comprennent au moins une carte électronique.
- il comprend des moyens d'alarme configurés pour se déclencher lorsqu'une valeur d'indice AOI ou d'indice moyen $AOI_{moy}$ est inférieure à un seuil donné, de préférence lorsque AOI < 0,75 (i.e. < 75%) ou $AOI_{moy}$ < 0,75 (i.e. < 75%).
- les moyens d'alarme comprennent une alarme sonore ou visuelle.
- les moyens d'alarme comprennent une alarme visuelle et l'IGU est configurée pour afficher ladite alarme visuelle, par exemple un voyant lumineux ou analogue.

**EP 3 639 735 B1**

- l'IGU comprend un écran digital (i.e. numérique), de préférence un écran tactile.
- l'écran comprend plusieurs touches activant différentes fonctions et/ou plusieurs zones ou fenêtres d'affichage.
- l'écran est du type à affichage en couleurs.
- alternativement, l'écran est du type à affichage en noir et blanc ou alors permet un passage d'un affichage en couleurs à un affichage en noir et blanc de manière à opérer des économies d'énergie.
- il comprend des moyens de mémorisation pour mémoriser (i.e. enregistrer) les valeurs de teneur en $CO_2$, les valeurs de teneur maximale en $CO_2$ (Vmax) et /ou les valeurs de teneur minimale en $CO_2$ (Vmin).
- il comprend des moyens de mémorisation coopérant avec les moyens de traitement de signal.
- les moyens de mémorisation comprennent une mémoire flash ou de type disque dur
- il comprend des moyens de mémorisation pour mémoriser (i.e. enregistrer) les valeurs d'indice AOI d'ouverture des voies respiratoires ou d'indice moyen $AOI_{moy}$.
- selon un premier mode de réalisation, il est choisi parmi les appareils de monitorage cardiaque, à savoir les moniteurs cardiaques ou les moniteurs/défibrillateurs cardiaques, pouvant mesurer et afficher des mesures d'un ou plusieurs paramètres physiologiques ou signes vitaux du patient, notamment cardiaques, par exemple l'activité électrique cardiaque, i.e. électrocardiogramme (ECG), et/ou la fréquence cardiaque du patient, qui sont mesurées par des électrodes placées sur le patient ; la saturation en oxygène ($SpO_2$) du patient, mesurée via un capteur transcutané ; la température via un capteur de température en contact avec le corps du patient, et/ou la pression artérielle (PA) grâce à un brassard de mesure de pression artérielle.
- il est un appareil de monitorage cardiaque comprenant des moyens de mesure de l'activité cardiaque sont configurés pour opérer des mesures en continu, en particulier plusieurs électrodes venant se placer sur le corps du patient. De préférence, les électrodes sont reliées électriquement aux moyens de traitement de signal et leur transmettent des signaux cardiaques, c'est-à-dire des signaux de mesure de l'activité cardiaque du patient, en particulier l'activité électrique du cœur du patient.
- les moyens de traitement de signal sont configurés pour traiter les signaux cardiaques provenant des moyens de mesure de l'activité cardiaque du patient.
- l'IGU est configurée pour afficher l'activité cardiaque du patient correspond aux signaux cardiaques traités, sous forme d'au moins une valeur numérique ou d'une représentation graphique, en particulier une courbe.
- les moyens de mesure de l'activité cardiaque du patient sont configurés pour mesurer la fréquence cardiaque, le rythme cardiaque et/ou l'activité électrique cardiaque (ECG) du patient.
- il comprend en outre des moyens de mesure de la saturation en oxygène ($SpO_2$) du patient, par exemple un capteur transcutané.
- il comprend en outre des moyens de mesure de la température corporelle du patient, par exemple un capteur de température en contact avec le corps du patient.
- il comprend en outre des moyens de mesure de la pression artérielle (PA), par exemple un brassard de mesure de pression artérielle.
- les moyens de mesure de l'activité cardiaque du patient sont conçus pour pouvoir délivrer au moins un choc électrique au patient, via une ou des électrodes apposées sur le patient.
- de façon alternative, selon un deuxième mode de réalisation, il est choisi parmi les appareils de ventilation assistée, i.e. les ventilateurs médicaux, comprenant une source de gaz respiratoire, notamment d'air.
- la source de gaz respiratoire est une micro-soufflante motorisée, encore appelée turbine ou compresseur, délivrant de l'air, éventuellement enrichi en oxygène.
- de façon générale, le $CO_2$ est produit par le patient, c'est-à-dire qu'il est observable lors de l'expiration du patient ou à l'inspiration suivante lorsqu'il s'agit de $CO_2$ réinhalé.
- les moyens de mesure de teneur en $CO_2$ sont agencés de manière à opérer des mesures de concentration en $CO_2$ dans un conduit de gaz reliant fluidiquement l'appareil de monitorage et/ou d'assistance respiratoire au patient.
- les moyens de mesure de teneur en $CO_2$ sont reliés électriquement aux moyens de traitement de signal.
- la source de gaz respiratoire est en communication fluidique avec un conduit de gaz servant à véhiculer le gaz respiratoire vers le patient, i.e. jusqu'à une interface respiratoire.
- le conduit de gaz est en communication fluidique avec une interface respiratoire, encore appelée interface patient.
- l'interface respiratoire est une sonde d'intubation endotrachéale, un masque facial ou un masque laryngé, aussi appelé dispositif supra glottique, ou tout dispositif adéquat d'administration de gaz.
- l'interface respiratoire est de préférence une sonde d'intubation endotrachéale, couramment appelée 'sonde trachéale'.
- les moyens de mesure de teneur en $CO_2$ sont en amont et à proximité immédiate de l'interface respiratoire, c'est-à-dire proche de la bouche du patient.
- selon un premier mode de réalisation, les moyens de mesure de teneur en $CO_2$ sont agencés sur une pièce de jonction agencée en amont de l'interface respiratoire, de préférence entre l'interface respiratoire et l'extrémité aval du conduit de gaz, en particulier entre l'interface respiratoire et une pièce en Y comprenant des passages internes de gaz.

- de préférence, les moyens de mesure de teneur en $CO_2$ sont agencés sur une pièce de jonction comprenant un passage interne de gaz.
- selon un deuxième mode de réalisation, les moyens de mesure de teneur en $CO_2$ sont agencés dans l'appareil, c'est-à-dire dans la carcasse de l'appareil, en étant reliés, via un conduit de prélèvement de gaz ou analogue, à un site de prélèvement de gaz situé en amont et à proximité immédiate de l'interface respiratoire.
- en particulier, les moyens de mesure de teneur en $CO_2$ sont reliés fluidiquement à un site de prélèvement de gaz porté par une pièce de jonction, en particulier agencée entre l'interface respiratoire et le conduit de gaz, typiquement entre l'interface respiratoire et une extrémité aval dudit conduit de gaz.
- la pièce de jonction est raccordée fluidiquement entre la pièce de raccordement intermédiaire, c'est-à-dire une pièce en Y, et l'interface respiratoire, typiquement une sonde trachéale ou un masque.
- il comprend un circuit patient comprenant une branche inspiratoire permettant d'acheminer du gaz vers le patient.
- le circuit patient comprend en outre une branche expiratoire permettant d'évacuer le gaz expiré par le patient.
- la branche inspiratoire, la branche expiratoire et l'interface respiratoire sont en communication fluidique entre elles.
- la branche inspiratoire, la branche expiratoire et l'interface patient sont connectées fluidiquement et/ou mécaniquement, directement ou indirectement, à la pièce de raccordement intermédiaire, en particulier une pièce en Y.
- les moyens de mesure de teneur en $CO_2$ sont agencés de manière à opérer des mesures de concentration en $CO_2$ à l'entrée de la branche expiratoire ou à la sortie de la branche inspiratoire du circuit de gaz.
- la branche expiratoire communique fluidiquement avec l'atmosphère pour évacuer tout ou partie du gaz expiré par le patient, en particulier le gaz riche en $CO_2$.
- la branche inspiratoire et/ou la branche expiratoire comprennent des tuyaux flexibles.
- de préférence, tout ou partie du conduit de gaz formant tout ou partie de la branche inspiratoire du circuit de gaz est un tuyau flexible.
- il comprend des moyens de pilotage configurés pour commander la source de gaz respiratoire et délivrer le gaz respiratoire selon des cycles ventilatoires successifs.
- chaque cycle ventilatoire comprend une phase BP pendant laquelle le gaz est délivré par la micro-soufflante à une pression dite basse ou basse pression (BP), et une phase HP pendant laquelle le gaz est délivré par la micro-soufflante à une pression dite haute ou haute pression (HP), avec HP > BP.
- la micro-soufflante est pilotée pour fournir du gaz à une pression basse (BP) comprise entre 0 et 20 cm d'eau, de préférence entre 0 et 15 cm d'eau, préférentiellement encore 0 et 10 cm d'eau.
- la micro-soufflante est pilotée pour fournir du gaz à une pression haute (HP) comprise entre 5 et 60 cm d'eau, de préférence entre 5 et 45 cm d'eau, préférentiellement encore 5 et 30 cm d'eau (avec HP > BP).
- la phase BP a une durée supérieure à la phase HP.
- la phase BP a une durée comprise entre 2 et 10 secondes, typiquement de l'ordre de 3 à 6 secondes.
- la phase HP a une durée comprise entre 0,5 et 3 secondes, typiquement de l'ordre de 1 à 2 secondes.
- la période de temps donnée (dt) est de plusieurs secondes.
- la période de temps (dt) est comprise entre 2 et 10 secondes, typiquement de l'ordre de 3 à 6 secondes.
- la période de temps (dt) correspond à la durée de la phase BP de chaque cycle ventilatoire.
- la durée totale d'un cycle ventilatoire est comprise entre 3 et 10 secondes.
- la période de temps (dt) donnée englobe plusieurs durées de compressions thoraciques et de relâchements successifs, typiquement entre 5 et 12 compressions thoraciques.
- il comprend une source de courant électrique, par exemple une batterie ou analogue, de préférence une batterie rechargeable.
- il comprend une carcasse ou coque externe rigide.
- la carcasse rigide est formée en tout ou en partie de polymère.
- l'iGU est agencée dans l'une des parois de la carcasse.
- il comprend en outre une IHM ou interface homme-machine permettant d'entrer, modifier/ajuster, sélectionner ....un ou des paramètres ventilatoires, par exemple les pressions, les durées etc....
- l'IHM comprend des moyens de réglage, par exemple un ou des boutons, touches, curseurs ou analogues.

L'invention va maintenant être mieux comprise grâce à la description détaillée suivante, faite à titre illustratif mais non limitatif, en référence aux figures annexées parmi lesquelles :

- la Figure 1 schématise un appareil de monitorage et d'assistance respiratoire selon l'invention,
- la Figure 2 est un exemple d'une courbe de teneur en CO2 au fil du temps correspondant à des voies aériennes bien ouvertes,
- la Figure 3 est analogue à la Figure 2 mais correspondant à des voies aériennes un peu moins ouvertes que dans le cas de la Figure 2,

- la Figure 4 est analogue à la Figure 2 mais correspondant à des voies aériennes fermées ou peu ouvertes.

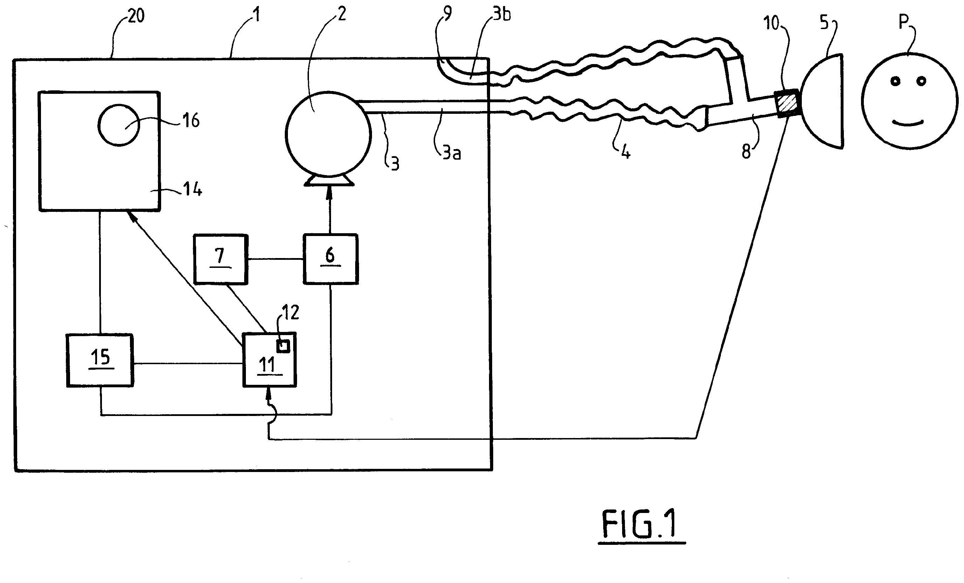

La Figure 1 est une représentation schématique d'un mode de réalisation d'un appareil 1 avec monitorage de la teneur en $CO_2$ selon l'invention, à savoir ici un ventilateur d'assistance respiratoire ou ventilateur médical servant à fournir un gaz respiratoire, typiquement de l'air ou de l'air enrichi en oxygène, à un patient pendant une réanimation cardio-pulmonaire (RCP), c'est-à-dire à une personne en arrêt cardiaque sur laquelle un secouriste pratique un massage cardiaque, c'est-à-dire une alternance de compressions thoraciques (CT) et de relâchements (R), c'est-à-dire de non-compressions thoraciques.

Cet appareil ou ventilateur 1 comprend une source 2 de gaz respiratoire, telle une micro-soufflante motorisée, qui est en communication fluidique avec un conduit de gaz 3 pour véhiculer et fournir le gaz respiratoire au patient P pendant la RCP, typiquement de l'air sous pression, via par exemple un conduit flexible 4 et une interface respiratoire 5 de distribution de gaz, tel un masque respiratoire facial ou laryngé, une sonde trachéale ou autre.

La source 2 de gaz respiratoire est commandée, c'est-à-dire pilotée, par des moyens de traitement de pilotage 6, notamment une carte électronique à microprocesseur ou analogue. Les moyens de pilotage 6 pilotent la source 2 de gaz respiratoire de manière à ce qu'elle délivre le gaz selon un ou plusieurs modes de ventilation prédéfinis et mémorisés au sein d'une mémoire 7, par exemple selon un mode ventilatoire « normal » correspondant à une ventilation d'un patient qui n'est pas ou plus en arrêt cardiaque et un mode ventilatoire « RCP » correspondant à une ventilation d'un patient qui est en arrêt cardiaque et sur lequel un secouriste entame ou pratique une RCP.

Par exemple, en mode de ventilation dédié à la RCP, la source 2 de gaz respiratoire est pilotée pour fournir de l'air selon un cycle ventilatoire comportant plusieurs niveaux de pression ou de type « BiPAP », en particulier 2 niveaux de pression comprenant un niveau de pression basse, par exemple une pression basse (BP) comprise entre environ 0 cm $H_2O$ et 15 cm $H_2O$, et un niveau de pression haute, par exemple une pression haute (HP) comprise entre environ 7 cm $H_2O$ et 40 cm $H_2O$. Le gaz est délivré alternativement entre ces deux niveaux de pression (BP, HP) pendant toute la réalisation de la RCP par le secouriste, c'est-à-dire pendant que le secouriste opère les CT et relâchements. La durée ($D_{BP}$) de fourniture du gaz à pression basse (BP) par la micro-soufflante 1 est comprise entre 2 et 10 secondes, typiquement de l'ordre de 3 à 6 secondes, alors que la durée ($D_{HP}$) de fourniture du gaz à pression haute (HP) est inférieure à 3 secondes, par exemple de l'ordre de 0,5 à 1,5 secondes.

La micro-soufflante 1 du ventilateur génère deux niveaux de pression, à savoir un niveau de pression haute (i.e PH) et un niveau de pression basse (i.e PB). Le massage cardiaque alternant les phases de compressions thoraciques (CTs) et de relâchements génère des pics de pression qui viennent se superposer aux cycles de pression du ventilateur. Il en résulte des pics de pression au niveau des plateaux hauts (i.e. à PH) et bas (i.e. à BP) qui reflètent les CTs avec augmentation de pression puisque le thorax s'affaisse sous la pression des CTs opérées par le secouriste, et des relâchements avec baisse de pression puisque le thorax remonte en l'absence de CT.

L'air fourni par la micro-soufflante 2 est acheminé par le conduit de gaz 3 qui forme tout ou partie de la branche inspiratoire 3a du circuit patient du ventilateur 1.

Le conduit de gaz 3 est en communication fluidique avec l'interface respiratoire 5, via le tuyau flexible 4, de manière à lui fournir l'air sous pression provenant de la micro-soufflante 2. Le conduit de gaz 2 vient se raccorder à l'interface respiratoire 5 par l'intermédiaire d'une pièce de raccordement intermédiaire 8, à savoir ici une pièce en Y. Cette pièce de raccordement intermédiaire 8 en Y comprend des passages de gaz internes et est par ailleurs raccordée à une branche expiratoire 3b du circuit patient du ventilateur 1 de manière à pouvoir recueillir et convoyer les gaz riches en $CO_2$ expirés par le patient P et les évacuer vers l'atmosphère (en 9).

Il est également prévu des moyens de mesure 10 de teneur en $CO_2$, appelés capteur de $CO_2$ 10 ou capnomètre, conçus pour opérer des mesures de concentration en $CO_2$ dans les gaz expirés par le patient P et fournir des signaux de mesure de teneur en $CO_2$ à des moyens de traitement de signal 11 où ces signaux de mesure peuvent être traités, notamment par un (ou des) algorithme de calcul ou analogue.

Dans le mode de réalisation de la Figure 1, le capteur de $CO_2$ 10 est agencé près de la bouche du patient P en configuration « flux principal » (*mainstream*), c'est-à-dire en amont et à proximité immédiate de l'interface respiratoire 5, préférentiellement entre la pièce en Y 8 et l'interface respiratoire 5, e.g. une sonde trachéale.

Toutefois, le capteur de $CO_2$ 10 peut aussi être agencé en configuration « flux dérivé » (*sidestream*), par exemple dans la carcasse 20 de l'appareil d'assistance respiratoire 1 et est relié, via une ligne de prélèvement de gaz, telle une tubulure ou analogue, à un site de prélèvement de gaz situé en amont et à proximité immédiate de l'interface respiratoire 5, par exemple sur la pièce de jonction 18. Cette ligne de prélèvement de gaz permet d'y prélever du gaz et l'acheminer ensuite jusqu'au capteur de $CO_2$ 10.

Le capteur de $CO_2$ 10 opère des mesures en continu de la concentration en $CO_2$ dans les gaz expirés par le patient P, en particulier le gaz circulant au travers de la pièce Y 8, lequel gaz est enrichi en $CO_2$ lors de son passage dans les poumons du patient P où s'effectue des échanges gazeux.

Les signaux de mesure de teneur en $CO_2$ sont ensuite transmis par le capteur de $CO_2$ 10, par liaison électrique 13 ou analogues, notamment filaire ou autre, aux moyens de traitement de signal 11 qui comprennent préférentiellement une carte électronique 12 à microprocesseur, de préférence à microcontrôleur, mettant en œuvre un (ou plusieurs) algorithme.

Préférentiellement, les moyens de traitement de signal 11 sont reliés électriquement aux moyens de mémorisation 7, par exemple une carte mémoire ou analogue, de manière à pouvoir y enregistrer tout ou partie des valeurs de teneur en $CO_2$ mesurées au fil du temps, en particulier pendant les CTs.

Il est à noter que, selon le mode de réalisation choisi, les moyens de traitement de signal 11 et les moyens de pilotage 6 peuvent être confondus, agencés sur ou comprendre une même carte électronique, ou alors agencés ou comprendre des cartes électroniques distinctes.

Dans le cadre de la présente invention, le monitorage d'un indice AOI revêt une grande importance car il permet d'informer en temps réel, le secouriste de l'ouverture ou non des voies aériennes et donc si les CTs successives opérées par le secouriste génèrent du débit ventilatoire ou non chez le patient P.

Plus précisément, les moyens de traitement de signal 11 sont configurés pour :

a) déterminer au moins une valeur de teneur maximale en $CO_2$ (Vmax) et au moins une valeur de teneur minimale en $CO_2$ (Vmin), pendant au moins une durée (dt) d'au moins une compression thoracique (CT), et

b) calculer au moins un indice AOI d'ouverture des voies respiratoires tel que :

$$AOI = (Vmax-Vmin) \,/\, Vmax$$

De préférence, les moyens de traitement de signal 11 sont configurés pour :

i) déterminer plusieurs valeurs de teneur maximale en $CO_2$ (Vmax) et plusieurs valeurs de teneur minimale en $CO_2$ (Vmin), pendant les durées (dt) de n compressions thoraciques (avec n>1) successives,

ii) calculer les indices AOI d'ouverture successifs, comme ci-dessus, correspondants aux valeurs de teneur maximale en $CO_2$ (Vmax) et plusieurs valeurs de teneur minimale en $CO_2$ (Vmin) et

iii) calculer un indice moyen $AOI_{moy}$ à partir des indices AOI d'ouverture successifs obtenus pour les n compressions thoraciques, avec : $AOI_{moy} = \sum_{i=1}^{n} AOI(i) \,/n$ où : n est un nombre entier de CT, avec n > 1.

Les moyens de mémorisation 7 peuvent également enregistrer tout ou partie des valeurs d'indice AOI et d'indice moyen $AOI_{moy}$ calculées par les moyens de traitement de signal 11.

Plus généralement, il a été constaté en pratique que les indices AOI et préférentiellement $AOI_{moy}$ reflètent l'ouverture des voies respiratoires du patient, pendant le massage cardiaque, et permettent d'informer, en temps réel, le(s) secouriste(s) de l'ouverture ou non de ces voies aériennes. Cette information est très utile au secouriste pour savoir si les insufflations d'air qu'il pratique pendant le massage cardiaque sont efficaces ou non, c'est-à-dire si l'air arrive bien ou non jusqu'aux petites voies aériennes intra-pulmonaires.

A cette fin, l'appareil 1 de l'invention comprend en outre au moins une interface graphique utilisateur ou IGU 14, tel un écran digital (e.g. couleurs, noir et blanc, ou les deux), de préférence un écran tactile, qui est reliée électriquement aux moyens de traitement de signal 11, lesquels sont configurés pour transmettre à l'IGU 14, la (ou les) valeur d'indice AOI ou d'indice moyen $AOI_{moy}$ ayant été calculés comme expliqué ci-avant.

L'IGU 14 est configurée quant à elle pour afficher cette valeur d'indice AOI ou d'indice moyen $AOI_{moy}$. En d'autres termes, l'IGU 14 affiche l'indice sous forme soit d'une valeur numérique, de préférence exprimée sous forme d'un pourcentage, soit d'une (ou plusieurs) représentation graphique 16, ou les deux. Comme exemples de représentation graphique 16, on peut citer un pictogramme, c'est-à-dire un dessin ou analogue, un barre-graphe, une courbe, un « camembert », par exemple une icône ou analogue représentant des poumons dont la taille et/ou la couleur varie en fonction de la valeur de l'indice AOI ou $AOI_{moy}$ ayant été déterminée.

La représentation graphique 16 peut être différente en fonction de la valeur de l'indice affiché afin d'en faciliter l'interprétation ou la compréhension par le secouriste, en particulier avoir une taille proportionnelle à la valeur de l'indice à afficher et/ou une couleur différente selon la valeur de l'indice à afficher.

Par exemple, on peut afficher un dessin représentant des poumons :

- de petite taille, et par exemple de couleur rouge, pour des valeurs d'indice AOI ou $AOI_{moy}$ inférieures à une valeur-seuil préfixée, par exemple < 0,75 (ou 75%),
- de taille moyenne, et par exemple de couleur orange, pour des valeurs d'indice AOI ou $AOI_{moy}$ entre 0,75 et 0,9 (ou 75% à 90%), et
- de plus grande taille, et par exemple de couleur verte, pour des valeurs d'indice AOI ou $AOI_{moy}$ inférieures à une valeur-seuil haute préfixée, par exemple > 0,9 (ou 90%).

On peut aussi prévoir des moyens d'alarme sonore ou visuelle configurés pour se déclencher lorsqu'une valeur d'indice AOI ou d'indice moyen $AOI_{moy}$ est inférieure à un seuil d'alerte donné, de préférence lorsque AOI < 0,75 (i.e. < 75%) ou $AOI_{moy}$ < 0,75 (i.e. < 75%), de préférence une alarme visuelle et l'IGU 14 est configurée pour afficher ladite alarme visuelle.

Une source de courant électrique 15, telle une batterie rechargeable ou analogue, alimente, directement ou indirectement, en courant électrique les moyens de traitement de signal 11 et les moyens de pilotage 6, la micro-soufflante 2, l'IGU 14 et tout autre élément de l'appareil, notamment les moyens de mémorisation 7. La source de courant électrique 15 est préférentiellement agencée dans la carcasse 20 du ventilateur.

L'appareil 1 selon la présente invention est particulièrement adapté à une mise en œuvre pendant une réanimation cardio-pulmonaire (RCP) sur une personne (i.e. un patient) en arrêt cardio-pulmonaire, dans le cadre de laquelle un gaz respiratoire, tel de l'air sous pression, est fourni selon un cycle ventilatoire à plusieurs niveaux de pression à ladite personne subissant le massage cardiaque avec alternance de compressions thoraciques et de relâchements. Pour faciliter son transport par les secouristes, par exemple un médecin, un infirmier, un pompier ou analogue, le ventilateur de l'invention est préférentiellement agencé dans un sac de transport.

L'appareil 1 selon la présente invention peut être un ventilateur médical, comme décrit ci-avant, ou alors un moniteur ou un moniteur cardiaque /défibrillateur cardiaque permettant de suivre, en outre l'activité cardiaque du patient, notamment via des électrodes, et éventuellement de lui délivrer des chocs électriques.

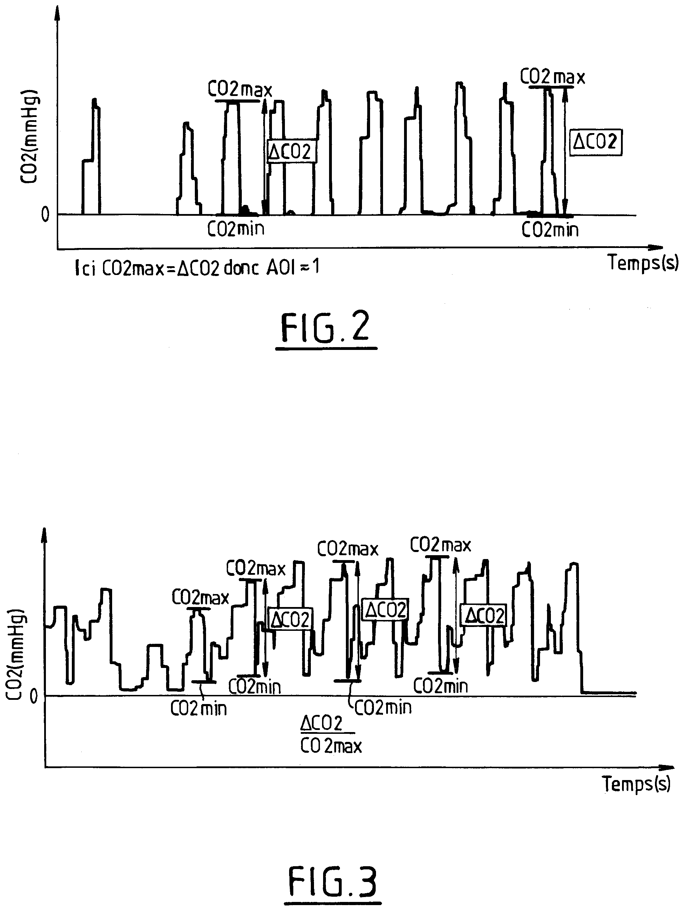

Les Figures 2 à 4 illustrent la présente invention.

Plus précisément, les Figures 2 et 3 sont des exemples de courbes de teneur en $CO_2$ au fil du temps correspondant à des voies aériennes totalement ouvertes (Fig. 2) ou presque totalement ouvertes (Fig. 3), c'est-à-dire très légèrement refermées par rapport à celles de la Figure 2.

Comme on le voit, ces courbes comprennent des pics de $CO_2$ qui se produisent lors des CTs opérées par le secouriste sur le patient P, pendant le massage cardiaque. La teneur en CO2 est exprimée ici en mmHg, i.e. pression partielle de CO2 (mmHg) dans le gaz; toutefois, la teneur en $CO_2$ pourrait être exprimée avec une autre grandeur, par exemple un % en volume, un % molaire ou autre.

Chaque pic de teneur en $CO_2$ se caractérise par une valeur haute (CO2max) et une valeur basse (CO2 min) correspondant aux valeurs maximale Vmax et minimale Vmin de teneur en $CO_2$ utilisées pour déterminer les indices AOI et $AOI_{moy}$. L'amplitude ΔCO2 de chaque pic correspond à la différence Vmax-Vmin pendant chaque CT réalisée pendant le massage cardiaque.

Sur la Figure 2, la valeur minimale de teneur en $CO_2$ est égale à 0 donc l'indice AOI est égal à 1, soit 100%, ce qui correspond à des voies aériennes totalement ouvertes pour lesquelles les transferts gazeux sont optimaux dans les poumons du patient.

Par ailleurs, sur la Figure 3, on voit que, dans ce cas, la valeur minimale de teneur en $CO_2$ est proche de 0, donc l'indice AOI est proche ou très proche de 1, par exemple de l'ordre de 90 à 99% environ, ce qui correspond à des voies aériennes presque totalement ouvertes pour lesquelles les transferts gazeux sont encore très bons.

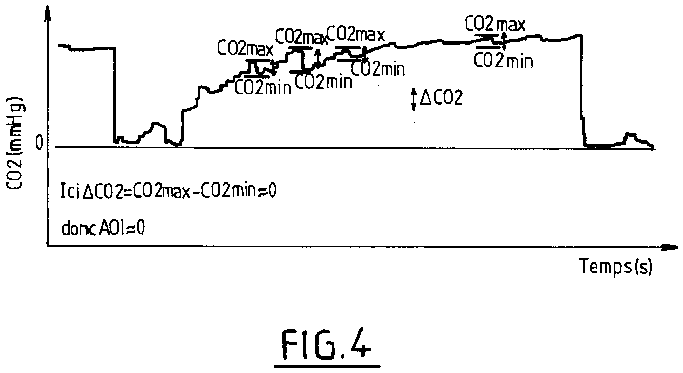

A l'inverse, on a illustré sur la Figure 4, une courbe de $CO_2$ obtenue pour un patient aux voies aériennes fermées pour lesquelles les échanges gazeux sont mauvais. Comme on le voit, dans ce cas, l'amplitude ΔCO2 de chaque pic, qui correspond à la différence Vmax-Vmin, est très faible, c'est-à-dire que les valeurs haute ($CO_2$max) et basse ($CO_2$min) sont proches, donc la différence (Vmax-Vmin) tend vers 0. On a dans ce cas, une valeur d'indice AOI faible, par exemple inférieure à 0,2 (i.e. < 20%).

On comprend immédiatement qu'en fournissant au secouriste cette valeur d'indice OAI pour chaque pic, c'est-à-dire chaque CT, ou alors une valeur moyennée OAImoy sur plusieurs CTs, c'est-à-dire correspond à plusieurs pics successifs, il peut avoir immédiatement une bonne idée de l'état d'ouverture des voies aériennes du patient et agir en conséquence.

Le fait de connaitre plus précisément l'état d'ouverture des voies aériennes, i.e. l'indice AOI selon l'invention, permet au secouriste d'avoir une meilleure information sur la ventilation dite « efficace » du patient, c'est-à-dire la quantité de ventilation qui arrive jusqu'aux alvéoles et qui participe donc aux échanges gazeux à travers la membrane alvéolo-capillaire du patient. En effet, c'est cette ventilation dite « efficace » du patient qui permet de ré-oxygéner efficacement le sang du patient et de l'épurer du $CO_2$ qu'il contient et ce, par diffusion au travers de la membrane alvéolo-capillaire des poumons du patient.

En connaissant cet indice AOI, l'utilisateur, typiquement le secouriste, peut décider d'ajuster la ventilation en modifiant tout ou partie des paramètres ventilatoires, lorsqu'il constate qu'elle n'est pas assez efficace, c'est-à-dire que la réoxygénation du sang du patient n'est pas suffisante.

En d'autres termes, grâce à la connaissance de l'indice AOI de l'invention, l'utilisateur peut opérer différents réglages du dispositif médical, en particulier du ventilateur servant à fournir du gaz respiratoire au patient, afin d'opérer une ventilation la plus efficace possible pour le patient. Cette information sur l'état d'ouverture des voies aériennes permet aussi au secouriste de prendre des décisions thérapeutiques, notamment sur la poursuite ou l'arrêt de la réanimation cardiopulmonaire (RCP) réalisée sur le patient.

**Revendications**

1. Appareil (1) de monitorage et/ou d'assistance respiratoire, utilisable pendant une réanimation cardio-pulmonaire (RCP) comprenant des compressions thoraciques successives de durée (dt) réalisées sur le patient et des relâchements, comprenant :

   - des moyens de mesure de teneur en $CO_2$ (10) pour opérer des mesures de concentration en $CO_2$ produit par le patient pendant la réanimation cardio-pulmonaire (RCP), et fournir des signaux de mesure de teneur en $CO_2$ à des moyens de traitement de signal (11),
   - des moyens de traitement de signal (11) configurés pour traiter les signaux de mesure de teneur en $CO_2$ provenant des moyens de mesure de teneur en $CO_2$ (10), et
   - au moins une interface graphique utilisateur (IGU) (14),

   **caractérisé en ce que** les moyens de traitement de signal (11) sont configurés pour :

   a) déterminer au moins une valeur de teneur maximale en $CO_2$ (Vmax) et au moins une valeur de teneur minimale en $CO_2$ (Vmin), pendant au moins une durée (dt) d'au moins une compression thoracique (CT), et
   b) calculer au moins :

   - un indice AOI d'ouverture des voies respiratoires tel que :

   $$AOI = (Vmax\text{-}Vmin) \, / \, Vmax$$

   ou
   - un indice moyen $AOI_{moy}$ à partir de plusieurs indices AOI d'ouverture successifs obtenus pendant les durées (dt) de n compressions thoraciques (avec n>1) successives, et

   c) transmettre à ladite interface graphique utilisateur (14), au moins une valeur d'indice AOI ou au moins une valeur d'indice moyen $AOI_{moy}$, et
   - l'interface graphique utilisateur (14) est configurée pour afficher ladite au moins une valeur d'indice AOI ou d'indice moyen $AOI_{moy}$, ou une représentation graphique (16) de ladite au moins une valeur d'indice AOI ou d'indice moyen $AOI_{moy}$.

2. Appareil selon la revendication précédente, **caractérisé en ce que** les moyens de traitement de signal (11) sont configurés pour :

   i) déterminer plusieurs valeurs de teneur maximale en $CO_2$ (Vmax) et plusieurs valeurs de teneur minimale en $CO_2$ (Vmin), pendant les durées (dt) de n compressions thoraciques (avec n>1) successives,
   ii) calculer les indices AOI d'ouverture successifs correspondants auxdites plusieurs valeurs de teneur maximale en $CO_2$ (Vmax) et plusieurs valeurs de teneur minimale en $CO_2$ (Vmin) et
   iii) calculer un indice moyen $AOI_{moy}$ à partir des indices AOI d'ouverture successifs obtenus pour les n compressions thoraciques.

3. Appareil selon l'une des revendications précédentes, **caractérisé en ce que** les moyens de traitement de signal (11) sont configurés pour calculer un indice moyen $AOI_{moy}$ à partir des indices AOI d'ouverture successifs obtenus pour n compressions thoraciques, tel que: $AOI_{moy} = \sum_{i=1}^{n} AOI(i) \, / n$ où : n est un nombre entier de CT, avec n > 1.

4. Appareil selon l'une des revendications précédentes, **caractérisé en ce que** l'interface graphique utilisateur (14) est configurée pour afficher ladite au moins une valeur d'indice AOI ou d'indice moyen $AOI_{moy}$ exprimée sous forme d'une valeur numérique, en particulier d'un pourcentage (%).

5. Appareil selon l'une des revendications 1 à 3, **caractérisé en ce que** l'IGU (14) est configurée pour afficher au moins une valeur d'indice AOI ou d'indice moyen $AOI_{moy}$ sous forme d'une représentation graphique (16) choisie parmi un pictogramme, une courbe, un barre-graphe ou un « camembert ».

6. Appareil selon l'une des revendications précédentes, **caractérisé en ce que** les moyens de mesure de teneur en $CO_2$ (10) comprennent un capnomètre, de préférence les moyens de mesure de teneur en $CO_2$ (10) sont configurés pour opérer des mesures de teneur en $CO_2$ en continu.

7. Appareil selon l'une des revendications précédentes, **caractérisé en ce que** les moyens de traitement de signal (11) comprennent au moins un microprocesseur (12), en particulier mettant en œuvre au moins un algorithme.

8. Appareil selon l'une des revendications précédentes, **caractérisé en ce qu'**il comprend des moyens d'alarme configurés pour se déclencher lorsqu'une valeur d'indice AOI ou d'indice moyen $AOI_{moy}$ est inférieure à un seuil donné, de préférence lorsque AOI < 0,75 (i.e. < 75%) ou $AOI_{moy}$ < 0,75 (i.e. < 75%).

9. Appareil selon la revendication 8, **caractérisé en ce que** les moyens d'alarme comprennent une alarme sonore ou visuelle, de préférence une alarme visuelle et l'IGU (14) est configurée pour afficher ladite alarme visuelle.

10. Appareil selon l'une des revendications précédentes, **caractérisé en ce que** l'IGU (14) comprend un écran digital, de préférence un écran tactile.

11. Appareil selon l'une des revendications précédentes, **caractérisé en ce qu'**il est choisi parmi les appareils de ventilation assistée (i.e. ventilateurs médicaux) comprenant une source (2) de gaz respiratoire, en particulier une micro-soufflante.

12. Appareil selon l'une des revendications 1 à 10, **caractérisé en ce qu'**il est choisi parmi les moniteurs cardiaques ou les moniteurs/défibrillateurs cardiaques.

**Patentansprüche**

1. Überwachungs- und/oder Beatmungsgerät (1), das bei einer kardiopulmonaren Reanimation (KPR) einsetzbar ist, die am Patienten durchgeführte aufeinanderfolgende Thoraxkompressionen einer Dauer (dt) und Entlastungen umfasst, umfassend:

   - Mittel zum Messen des $CO_2$-Gehalts (10) zum Durchführen von Messungen der Konzentration von $CO_2$, das bei der kardiopulmonaren Reanimation (KPR) vom Patienten erzeugt wird, und Bereitstellen der Messsignale für den $CO_2$-Gehalt an Signalverarbeitungsmittel (11),
   - Signalverarbeitungsmittel (11), die dazu konfiguriert sind, die Messsignale für den $CO_2$-Gehalt, die aus den Mitteln zum Messen des $CO_2$-Gehalts (10) stammen, zu bearbeiten, und
   - mindestens eine grafische Benutzeroberfläche (GUI) (14),
   **dadurch gekennzeichnet, dass** die Signalverarbeitungsmittel (11) zu Folgendem konfiguriert sind:

   a) Bestimmen mindestens eines Wertes des maximalen $CO_2$-Gehalts (Vmax) und mindestens eines Wertes des minimalen $CO_2$-Gehalts (Vmin) während mindestens einer Dauer (dt) von mindestens einer Thoraxkompression (TK), und
   b) Berechnen von mindestens:

      - einem Index AOI der Atemwegsöffnung wie etwa:

$$AOI = (Vmax - Vmin) / Vmax$$

      oder
      - einem mittleren Index $AOI_{mit}$ auf Grundlage mehrerer aufeinanderfolgender Öffnungsindizes AOI, die während der Dauer (dt) von n aufeinanderfolgenden Thoraxkompressionen (mit n>1) erhalten werden, und

c) Senden mindestens eines Wertes des Indexes AOI oder mindestens eines Wertes des mittleren Indexes AOI$_{mit}$ an die grafische Benutzeroberfläche (14), und

- die graphische Benutzeroberfläche (14) zum Anzeigen mindestens eines Wertes des Indexes AOI oder des mittleren Indexes AOI$_{mit}$ oder einer grafischen Darstellung (16) des mindestens eines Wertes des Indexes AOI oder des mittleren Indexes AOI$_{mit}$ konfiguriert ist.

2. Gerät nach dem vorherigen Anspruch, **dadurch gekennzeichnet, dass** die Signalverarbeitungsmittel (11) zu Folgendem konfiguriert sind:

i) Bestimmen mehrerer Werte des maximalen $CO_2$-Gehalts (Vmax) und mehrerer Werte des minimalen $CO_2$-Gehalts (Vmin) während der Dauer (dt) von n aufeinanderfolgenden Thoraxkompressionen (mit n>1),
ii) Berechnen der aufeinanderfolgenden Öffnungsindizes AOI, die den mehreren Werten des maximalen $CO_2$-Gehalts (Vmax) und den mehreren Werten des minimalen $CO_2$-Gehalts (Vmin) entsprechen, und
iii) Berechnen eines mittleren Indexes AOI$_{mit}$ auf Grundlage der aufeinanderfolgenden Öffnungsindizes AOI, die für die n Thoraxkompressionen erhalten wurden.

3. Gerät nach einem der vorhergehenden Ansprüche, **dadurch gekennzeichnet, dass** die Signalverarbeitungsmittel (11) konfiguriert sind zum Berechnen eines mittleren Indexes AOI$_{mit}$ auf Grundlage der aufeinanderfolgenden Öffnungsindizes AOI, die für n Thoraxkompressionen erhalten wurden, wie etwa: $\texttt{AOI}_{\texttt{mit}} \; = \; \sum_{i=1}^{n} AOI(i)/n$ wobei: n eine ganze Zahl von TK ist, mit n>1.

4. Gerät nach einem der vorhergehenden Ansprüche, **dadurch gekennzeichnet, dass** die grafische Benutzerschnittstelle (14) konfiguriert ist zum Anzeigen des mindestens einen Wertes des Indexes AOI oder des mittleren Indexes AOI$_{mit}$, ausgedrückt in Form eines Zahlenwertes, insbesondere eines Prozentsatzes (%).

5. Gerät nach einem der Ansprüche 1 bis 3, **dadurch gekennzeichnet, dass** die GUI (14) konfiguriert ist zum Anzeigen mindestens eines Wertes des Indexes AOI oder des mittleren Indexes AOI$_{mit}$ in Form einer grafischen Darstellung (16), die aus einem Piktogramm, einer Kurve, einem Balkendiagramm oder einem Kreisdiagramm ausgewählt ist.

6. Gerät nach einem der vorhergehenden Ansprüche, **dadurch gekennzeichnet, dass** die Mittel zum Messen des $CO_2$-Gehalts (10) ein Capnometer umfassen, vorzugsweise die Mittel zum Messen des $CO_2$-Gehalts (10) zum Durchführen kontinuierlicher Messungen des $CO_2$-Gehalts konfiguriert sind.

7. Gerät nach einem der vorhergehenden Ansprüche, **dadurch gekennzeichnet, dass** die Signalverarbeitungsmittel (11) mindestens einen Mikroprozessor (12) umfassen, der insbesondere mindestens einen Algorithmus ausführt.

8. Gerät nach einem der vorhergehenden Ansprüche, **dadurch gekennzeichnet, dass** er Alarmmittel umfasst, die dazu konfiguriert sind, dann auszulösen, wenn ein Wert des Indexes AOI oder des mittleren Indexes AOI$_{mit}$ kleiner ist als ein bestimmter Schwellenwert, vorzugsweise wenn AOI <0,75 (d. h. <75 %) oder AOI$_{mit}$ <0,75 (d. h. <75 %).

9. Gerät nach Anspruch 8, **dadurch gekennzeichnet, dass** die Alarmmittel einen akustischen oder visuellen Alarm umfassen, vorzugsweise einen visuellen Alarm, und die GUI (14) zum Anzeigen des visuellen Alarms konfiguriert ist.

10. Gerät nach einem der vorhergehenden Ansprüche, **dadurch gekennzeichnet, dass** die GUI (14) einen digitalen Bildschirm umfasst, vorzugsweise einen Tastbildschirm.

11. Gerät nach einem der vorhergehenden Ansprüche, **dadurch gekennzeichnet, dass** es aus Beatmungsgeräten (d. h. medizinischen Ventilatoren) ausgewählt ist, die eine Quelle (2) von Atemgas umfassen, insbesondere ein Mikrogebläse.

12. Gerät nach einem der Ansprüche 1 bis 10, **dadurch gekennzeichnet, dass** es aus Herzüberwachungsgeräten oder Herzüberwachungs-/-defibrillationsgeräten ausgewählt ist.

**Claims**

1. Monitoring and/or respiratory assistance apparatus (1) that can be used during a cardiopulmonary resuscitation (CPR) with successive chest compressions of duration (dt) performed on the patient and with relaxations, said apparatus comprising:

   - means (10) for measuring the $CO_2$ content in order to perform measurements of the concentration of $CO_2$ produced by the patient during the cardiopulmonary resuscitation (CPR), and to supply $CO_2$ content measurement signals to signal-processing means (11),
   - signal-processing means (11) configured to process the $CO_2$ content measurement signals originating from the $CO_2$ content measurement means (10), and
   - at least one graphical user interface (GUI) (14), **characterized in that** the signal-processing means (11) are configured to:

      a) determine at least one maximum $CO_2$ content value (Vmax) and at least one minimum $CO_2$ content value (Vmin), during at least one duration (dt) of at least one chest compression (CC), and
      b) calculate at least:

         - one airway opening index AOI such that:

$$AOI = (Vmax-Vmin) / Vmax$$

         or

         - a mean index $AOI_{mean}$ on the basis of several successive opening indices AOI obtained during the durations (dt) of n successive chest compressions (with n>1), and
         c) transmit to said graphical user interface (14) at least one index value AOI or at least one mean index value $AOI_{mean}$, and
         - the graphical user interface (14) is configured to display said at least one index value AOI or mean index value $AOI_{mean}$, or a graphical representation (16) of said at least one index value AOI or mean index value $AOI_{mean}$.

2. Apparatus according to the preceding claim, **characterized in that** the signal-processing means (11) are configured to:

   i) determine several maximum $CO_2$ content values (Vmax) and several minimum $CO_2$ content values (Vmin) during the durations (dt) of n successive chest compressions (with n>1),
   ii) calculate the successive opening indices AOI corresponding to said several maximum $CO_2$ content values (Vmax) and several minimum $CO_2$ content values (Vmin), and
   iii) calculate a mean index $AOI_{mean}$ on the basis of the successive opening indices AOI obtained for the n chest compressions.

3. Apparatus according to either of the preceding claims, **characterized in that** the signal-processing means (11) are configured to calculate a mean index $AOI_{mean}$ on the basis of the successive opening indices AOI obtained for n chest compressions, such that: $AOI_{mean} = \sum_{i=1}^{n} AOI(i) / n$ where: n is an integer of CC, with n > 1.

4. Apparatus according to one of the preceding claims, **characterized in that** the graphical user interface (14) is configured to display said at least one index value AOI or mean index value $AOI_{mean}$ expressed in the form of a numerical value, in particular a percentage (%).

5. Apparatus according to one of Claims 1 to 3, **characterized in that** the GUI (14) is configured to display at least one index value AOI or mean index value $AOI_{mean}$ in the form of a graphical representation (16) chosen from a pictogram, a curve, a bar graph or a pie chart.

6. Apparatus according to one of the preceding claims, **characterized in that** the means (10) for measuring the $CO_2$ content comprise a capnometer, and preferably the means (10) for measuring the $CO_2$ content are configured to

perform $CO_2$ content measurements continuously.

7. Apparatus according to one of the preceding claims, **characterized in that** the signal-processing means (11) comprise at least one microprocessor (12), in particular using at least one algorithm.

8. Apparatus according to one of the preceding claims, **characterized in that** it comprises alarm means configured to trigger when an index value AOI or mean index value $AOI_{mean}$ is below a given threshold, preferably when AOI < 0.75 (i.e. < 75%) or $AOI_{mean}$ < 0.75 (i.e. < 75%).

9. Apparatus according to Claim 8, **characterized in that** the alarm means comprise an acoustic or visual alarm, preferably a visual alarm, and the GUI (14) is configured to display said visual alarm.

10. Apparatus according to one of the preceding claims, **characterized in that** the GUI (14) comprises a digital screen, preferably a touch screen.

11. Apparatus according to one of the preceding claims, **characterized in that** it is chosen from among the assisted ventilation apparatuses (i.e. medical ventilators) comprising a source (2) of respiratory gas, in particular a micro-blower.

12. Apparatus according to one of Claims 1 to 10, **characterized in that** it is chosen from among cardiac monitors or cardiac monitors/defibrillators.

FIG.1

Ici CO2max = ΔCO2 donc AOI ≈ 1

## FIG.2

$$\frac{\Delta CO2}{CO2max}$$

## FIG.3

FIG.4

**EP 3 639 735 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- EP 3093498 A
- EP 2947328 A
- EP 2986856 A
- EP 2954213 A
- EP 2102504 A
- US 20160287170 A
- WO 14072981 A
- US 2016133160 A
- US 2012016279 A